Europäisches Patentamt

European Patent Office

Office européen des brevets

(19)

(11) Publication number: **0 322 149**
**A2**

## EUROPEAN PATENT APPLICATION

(12)

(21) Application number: 88311800.2

(22) Date of filing: 14.12.88

(51) Int. Cl.⁴: **C07D 471/04 , A61K 31/435 ,**
**//(C07D471/04,235:00,221:00)**

(30) Priority: 22.12.87 US 136899

(43) Date of publication of application:
**28.06.89 Bulletin 89/26**

(84) Designated Contracting States:
**AT BE CH DE ES FR GB GR IT LI LU NL SE**

(71) Applicant: **ELI LILLY AND COMPANY**
**Lilly Corporate Center**
**Indianapolis Indiana 46285(US)**

(72) Inventor: **Cuff, George William**
**2126 East 61st Place**
**Indianapolis Indiana 46220(US)**
Inventor: **Greene, James Michael**
**607 West Ralston Road**
**Indianapolis Indiana 46217(US)**

(74) Representative: **Tapping, Kenneth George et al**
**Erl Wood Manor**
**Windlesham Surrey, GU20 6PH(GB)**

(54) Hydrates of an inotropic agent.

(57) The present invention provides new compounds; namely, 2-[2-methoxy-4-(methylsulfinyl)phenyl]-1(3)H-imidazo[4,5-c]pyridine hydrochloride monohydrate, its individual polymorphs, and 2-[2-methoxy-4-(methylsulfinyl)phenyl]-1(3)H-imidazo[4,5-c]pyridine hydrochloride dihydrate, their methods of use, and pharmaceutical formulations. Also provided are processes for preparing the new compounds.

EP 0 322 149 A2

Xerox Copy Centre

# HYDRATES OF AN INOTROPIC AGENT

This invention relates to novel hydrates of the inotropic agent 2-[2-methoxy-4-(methylsulfinyl)phenyl]-1-(3)H-imidazo[4,5-c]pyridine hydrochloride, more particularly to novel crystalline hydrates of that compound, their methods of use, pharmaceutical formulations and processes of preparation.

European Patent Application No. 93,593 and United States Patent No. 4,603,139 teach 2-[2-methoxy-4-(methylsulfinyl)phenyl]-1(3)H-imidazo[4,5-c]pyridine as a potent inotropic agent. In the production of such pharmaceutical compounds it is important to have a crystalline form which is stable for long periods of time under inexpensive storage conditions. The present invention provides just such stable crystalline forms.

According to the present invention there are provided hydrates of 2-[2-methoxy-4-(methylsulfinyl)-phenyl]-1(3)H-imidazo[4,5-c]pyridine hydrochloride; namely, 2-[2-methoxy-4-(methylsulfinyl)phenyl]-1(3)H-imidazo[4,5-c]pyridine hydrochloride monohydrate and the corresponding 2-[2-methoxy-4-(methylsulfinyl)-phenyl]-1(3)H-imidazo[4,5-c]pyridine hydrochloride dihydrate. Preferred compounds of this invention are crystalline forms of the monohydrate and dihydrate. Crystalline monohydrate exists in three polymorphic states (referred to herein as States I, II and III) which have the x-ray powder diffraction patterns described in Tables 1, 2, and 3, respectively, below, while the dihydrate can be crystallized in a single crystalline form. The dihydrate, in particular, is highly stable, making it an especially advantageous crystalline form for making pharmaceutical formulations of 2-[2-methoxy-4-(methylsulfinyl)phenyl]-1(3)H-imidazo[4,5-c]pyridine hydrochloride.

The present invention provides 2-[2-methoxy-4-(methylsulfinyl)phenyl]-1(3)H-imidazo[4,5-c]pyridine hydrochloride monohydrate and processes for preparing it. The monohydrate can crystallize in three crystalline forms, each of which assays approximately 5% (weight %) water by Karl Fisher analysis. The crystalline monohydrate polymorphs are characterized by the x-ray powder diffraction patterns listed in Tables 1, 2, and 3 below. The diffraction pattern was obtained with nickel-filtered copper radiation (Cu:Ni) of wavelength $\lambda$ = 1.54056Å. The interplanar spacings are in the column headed by "d" and the relative intensities in the column "$I/I_1$".

Polymorphic State I

Table 1

| d | $I/I_1$ |
|---|---|
| 10.07 | .35 |
| 6.37 | .11 |
| 5.86 | .22 |
| 5.68 | .04 |
| 4.97 | .48 |
| 4.55 | .05 |
| 4.43 | .02 |
| 4.25 | .13 |
| 4.19 | .03 |
| 3.96 | .36 |
| 3.60 | 1.00 |
| 3.33 | .49 |
| 2.89 | .36 |
| 2.34 | .14 |
| 2.12 | .06 |
| 2.03 | .06 |

Polymorphic State II

Table 2

| d | I/I$_1$ |
|---|---|
| 14.12 | .11 |
| 10.68 | .11 |
| 6.96 | .71 |
| 5.95 | .11 |
| 5.03 | .22 |
| 4.83 | .56 |
| 4.67 | .14 |
| 3.97 | .38 |
| 3.86 | .06 |
| 3.75 | .17 |
| 3.51 | 1.00 |
| 3.27 | .16 |
| 3.10 | .50 |
| 2.86 | .08 |
| 2.68 | .06 |
| 2.34 | .25 |
| 2.00 | .11 |

Polymorphic State III

Table 3

| d | I/I$_1$ |
|---|---|
| 13.86 | .08 |
| 10.61 | .11 |
| 10.04 | .06 |
| 7.60 | .04 |
| 6.93 | .52 |
| 6.37 | .07 |
| 5.89 | .25 |
| 5.30 | .09 |
| 4.98 | .30 |
| 4.81 | .66 |
| 4.64 | .25 |
| 4.25 | .20 |
| 3.96 | 1.00 |
| 3.74 | .32 |
| 3.60 | .80 |
| 3.50 | 1.00 |
| 3.34 | .33 |
| 3.17 | .16 |
| 3.09 | .89 |
| 2.86 | .13 |
| 2.67 | .15 |

The monohydrate crystalline forms can be prepared from 2-[2-methoxy-4-(methylsulfinyl)phenyl]-1(3)H-imidazo[4,5-c]pyridine hydrochloride, which is a known compound and can be prepared according to procedures disclosed in King; United States Patent No. 4,603,139, issued July 29, 1986, and Jonas; United Patent No. 4,616,090, issued October 7, 1986, both of which are herein incorporated by reference. A monohydrate crystalline form having the x-ray powder diffraction pattern shown in Table 1 can be obtained by preparing a slurry consisting of the hydrochloride salt in an alcohol solvent. Suitable alcohol solvents include solvents such as methanol, ethanol, n-propanol, isopropanol or the like. The Polymorphic State I is obtained by intimately mixing using any conventional mixing technique, for example by mechanically shaking, the solid and liquid phases of the slurry for a period of about 1 hour to about 24 hours. Optionally, the State I monohydrate crystal can then be isolated by any standard isolation technique, for example by vacuum filtration. One set of reaction conditions for preparing the crystalline monohydrate polymorph which has the x-ray powder diffraction pattern shown in Table 1 is illustrated in Example 1.

The crystalline monohydrate polymorph which has the x-ray powder diffraction pattern shown in Table 2 can be obtained by substantially dissolving the hydrochloride salt in water. The temperature of the water used to dissolve the hydrochloride salt may vary from about room temperature to about 75° C. Once the salt is substantially dissolved, an alcohol, preferably isopropanol, counter-solvent is added. The counter-solvent is added in an amount sufficient to form an approximately 4:1 (v:v) to 10:1 (v:v) alcohol/water mixture. After the alcohol counter-solvent is added the State II monohydrate is crystallized by cooling the alcohol/water mixture as rapidly as possible to a temperature in the range of from about -20° C to about 10° C. Once the mixture is cooled to the desired temperature, the State II monohydrate can be isolated by any standard isolation technique, for example by vacuum filtration. If the isolated crystals are washed, a pure alcohol solvent wash solution should be used as the final wash solution. A specific set of reaction conditions for preparing the crystalline monohydrate polymorph which has the x-ray powder diffraction pattern shown in Table 2 is illustrated in Example 2.

A third crystalline monohydrate polymorph which has the x-ray powder diffraction pattern shown in Table 3 can be obtained by recrystallizing the monohydrate from a mixture of crystalline monohydrate and dihydrate compounds. The crystalline monohydrate and dihydrate mixture required to produce the State III monohydrate can be produced by dissolving 2-[2-methoxy-4-(methylsulfinyl)phenyl]-1(3)H-imidazo[4,5-c]-pyridine hydrochloride in water, cooling the solution prior to alcohol addition, and, while cooling the solution, adding an alcohol counter-solvent at a rate such that a substantial amount of crystallization occurs before the alcohol addition can be completed. The alcohol counter-solvent should be added in an amount sufficient to form a water/alcohol mixture ranging in composition from about 4:1 (v:v) alcohol/water to about 10:1 (v:v) alcohol/water. Alternatively, the monohydrate and dihydrate crystalline mixture can be prepared either by washing pure dihydrate crystals with a final wash solution composed of an alcohol solvent, or by drying pure dihydrate crystals at temperatures in excess of about 40° C.

The monohydrate/dihydrate mixture produced by the methods detailed above is then converted to a crystalline monohydrate State III polymorph by substantially dissolving the mixture in water. Once the mixture is substantially dissolved, an alcohol counter-solvent is added in an amount sufficient to form an alcohol/water mixture ranging in composition from about 4:1 (v:v) to about 10:1 (v:v) alcohol/water. After the counter-solvent is added, the desired monohydrate form is crystallized by externally cooling the alcohol/water mixture as rapidly as possible to a temperature in the range of from about -20° C to about 10° C. The crystals produced are then isolated using standard isolation techniques. The isolated crystals can optionally be washed with pure alcohol solvent. A specific set of parameters for a process for preparing the State III crystalline monohydrate polymorph is set forth in Example 3.

The present invention also provides 2-[2-methoxy-4-(methylsulfinyl)phenyl]-1(3)H-imidazo[4,5-c]pyridine hydrochloride dihydrate and processes for preparing it. The crystalline dihydrate assays approximately 10% (weight %) water by Karl Fisher analysis. The dihydrate is a preferred hydrate since it is the most stable crystalline form.

The crystalline dihydrate compound is a microcrystalline solid characterized by the x-ray powder diffraction pattern listed in Table 4 below. The diffraction pattern was obtained with nickel-filtered copper radiation (Cu:Ni) of wavelength $\lambda$ = 1.54056Å. The interplanar spacings are in the column headed by "d" and the relative intensities in the column "$I/I_1$".

Crystalline Dihydrate

4

Table 4

| d | $I/I_1$ |
|---|---|
| 13.05 | .05 |
| 11.67 | .24 |
| 9.23 | .11 |
| 7.60 | .05 |
| 7.27 | .25 |
| 6.57 | .16 |
| 5.86 | .45 |
| 5.25 | .09 |
| 4.88 | .16 |
| 4.15 | .28 |
| 3.90 | 1.00 |
| 3.62 | .20 |
| 3.49 | .56 |
| 3.35 | .07 |
| 3.24 | .46 |
| 3.18 | .42 |
| 3.07 | .08 |
| 2.99 | .10 |
| 2.93 | .05 |
| 2.62 | .18 |

The dihydrate can be prepared by two processes. One process involves converting 2-[2-methoxy-4-(methylsulfinyl)phenyl]-1(3)H-imidazo[4,5-c]pyridine hydrochloride monohydrate to the dihydrate form. The other process entails converting 2-[2-methoxy-4-(methylsulfinyl)phenyl]-1(3)H-imidazo[4,5-c]pyridine directly to the dihydrate.

The process for converting monohydrate to dihydrate may be conducted using any one of three procedures. One procedure consists of, first, dissolving the monohydrate in water or a water/alcohol or ketone organic solvent mixture, followed by the addition of more alcohol or ketone organic solvent. A preferred embodiment of this procedure entails the additional step of collecting the precipitated dihydrate.

More specifically, the procedure consists of substantially dissolving the monohydrate in water or a water/alcohol or ketone organic solvent mixture which is comprised of up to about 2 ml of organic solvent per ml of water. Acceptable alcohol or ketone organic solvents include alcohols such as methanol, ethanol, n-propanol, isopropanol and the like, and ketones such as acetone, methyl ethyl ketone and the like. Ethanol and isopropanol are preferred organic solvents, with isopropanol most preferred. The temperature of the water or water/organic solvent mixture may vary from about room temperature to about 75°C, with a preferred temperature being about 60°C.

After the dissolution step, additional alcohol or ketone organic solvent, as provided above, is added in order to crystallize the dihydrate. The organic solvent is added in an amount sufficient to form a water/organic solvent mixture ranging in composition from about 4 milliliters of organic solvent per milliliter of water to about 10 milliliters of organic solvent per milliliter of water. A preferred solution contains about 7.5 milliliters of organic solvent per milliliter of water. The water/organic solvent mixture also may optionally be seeded with 2-[2-methoxy-4-(methylsulfinyl)phenyl]-1(3)H-imidazo[4,5-c]pyridine hydrochloride dihydrate.

In order to obtain the dihydrate in high yield, the water/alcohol or ketone organic solvent mixture should be cooled to a temperature in the range of from about -10°C to about 25°C. Mixtures which require cooling to reach the desired temperature should be cooled slowly in order to ensure formation of dihydrate crystals rather than monohydrate crystals. These mixtures should be cooled to the desired temperature over a time period ranging from about 60 minutes to until crystallization is substantially complete. A preferred cooling period ranges from about 60 minutes to about 16 hours depending on the mixture's initial temperature and volume, as well as the final temperature desired. The greater the volume or initial temperature or the lower the desired final temperature, of course, the longer the cooling period required. Generally, the dihydrate is most readily produced if the mixture has an initial temperature of about 60°C and is then cooled in such a manner that most of the crystallization occurs between about 40°C and about 60°C.

Once the mixture is cooled to the desired temperature, the dihydrate can be recovered at once, or the mixture can be allowed to stir for several hours to enhance the yield of dihydrate. The crystalline 2-[2-methoxy-4-(methylsulfinyl)phenyl]-1(3)H-imidazo[4,5-c]-pyridine hydrochloride dihydrate product can be recovered, if desired, using any standard isolation technique, for example, gravity filtration, vacuum filtration, pressure filtration, centrifugal filtration, or centrifugation, with the most preferred method of isolation being vacuum filtration.

A second procedure for converting monohydrate to dihydrate entails preparing a slurry consisting of monohydrate mixed with water and an alcohol or ketone organic solvent, then contacting the solid phase with the liquid phase. Again, a preferred embodiment of this procedure entails the additional step of collecting the dihydrate.

The monohydrate slurry is prepared by adding 2-[2-methoxy-4-(methylsulfinyl)phenyl]-1(3)H-imidazo-[4,5-c]pyridine hydrochloride monohydrate to a mixture composed of water and an alcohol or ketone organic solvent in approximately a 40% to about a 95% (volume %) organic solvent ratio. A preferred mixture is composed of about 90% organic solvent. Suitable alcohol or ketone organic solvents for preparing the slurry include alcohols such as methanol, ethanol, n-propanol, isopropanol and the like, and ketones such as acetone, methylethyl ketone and the like. Isopropanol and acetone are preferred organic solvents, with acetone the most pre ferred solvent. The monohydrate is then added to the mixture in quantities sufficient to exceed the mixture's saturation point, thus providing a slurry.

The solid phase of the slurry is contacted with the liquid phase by any conventional means, for example, a pneumatic or electrically powered rotating impeller, a magnetically operated stirring rod, a mechanical shaker, a mechanical mixer, or by pulsing short bursts of air, nitrogen, or some other inert gas up through the solid phase of the slurry, for a period of about 1 to about 48 hours. After contacting the phases the resulting dihydrate product may be isolated, if desired, using any standard isolation technique as discussed in the first procedure for converting monohydrate to dihydrate set forth above. Vacuum filtration is a preferred isolation technique in the slurry process of the present invention.

A third procedure for converting monohydrate to dihydrate comprises exposing the monohydrate to a gas which has a relative humidity of about 50% relative humidity to about 95% relative humidity and a temperature in the range of from about 15°C to about 60°C. A preferred gas temperature is room temperature (approximately 24°C). Gases which may be used in the present procedure include inert gases such as nitrogen, argon, air and the like. Air is a preferred inert gas. The monohydrate is exposed to a gas which has a relative humidity and a temperature within the ranges noted above for a period of time sufficient to convert it to the dihydrate. In general this period of time will range from as short a period of time as 1 day to as long a period of time as four months depending on the relative humidity and temperature conditions chosen. A preferred period of time for exposing the monohydrate to the gas is from about two weeks to about three months.

The second process for preparing 2-[2-methoxy-4-(methylsulfinyl)phenyl]-1(3)H-imidazo[4,5-c]pyridine hydrochloride dihydrate entails direct preparation from 2-[2-methoxy-4-(methylsulfinyl)phenyl]-1(3)H-imidazo[4,5-c]pyridine. Specifically, the process comprises substantially dissolving 2-[2-methoxy-4-(methylsulfinyl)phenyl]-1(3)H-imidazo-[4,5-c]pyridine, or a weak acid salt thereof, in a water/alcohol or ketone organic solvent mixture, adding hydrochloric acid, and cooling the mixture to a temperature in the range of from about -15°C to about 10°C. Again, a preferred embodiment of this process entails the additional step of collecting the precipitated dihydrate.

The claimed process for directly preparing the dihydrate requires 2-[2-methoxy-4-(methylsulfinyl)-phenyl]-1(3)H-imidazo-[4,5-c]pyridine, or a weak acid salt of that compound, as starting material. Weak acid salts of the imidazopyridine compound, as defined in this application, are salts formed from acids which have a pKa of between about 3.0 to about 6.0. Such acids include lower alkanoic acids such as acetic acid, propionic acid, valeric acid and the like, other carboxylic acids such as formic acid, p-nitrobenzoic acid, benzoic acid, chloroacetic acid and the like, other organic acids such as 2,4-dinitrophenol and the like, and inorganic acids such as carbonic acid, nitric acid and the like. The imidazopyridine starting material and its weak acid salts are known in the literature and can be prepared according to procedures disclosed in Jonas; United States Patent No. 4,616,090, issued October 7, 1986, which is herein incorporated by reference. For a further disclosure of the imidazopyridine starting material and its weak acid salts, see European Patent Application No. 93,593.

The dihydrate is prepared by first, substantially dissolving the imidazopyridine, or its weak acid salt, in a water/alcohol or ketone organic solvent mixture. Suitable alcohol or ketone organic solvents include alcohols such as methanol, ethanol, n-propanol, isopropanol and the like, and ketones such as acetone, methyl ethyl ketone and the like. Isopropanol is the preferred solvent when directly preparing the dihydrate. The composition of the water/organic solvent mixture may vary from about 4:1 to about 10:1 (v:v) organic solvent/water. A preferred mixture consists of about a 7:1 (v:v) organic solvent/water mixture.

Once the imidazopyridine is substantially dissolved, hydrochloric acid is added. Hydrochloric acid may

EP 0 322 149 A2

be added in either liquid or gaseous form, although concentrated liquid hydrochloric acid (38% by weight hydrochloric acid) is preferred. The amount of hydrochloric acid added is not critical so long as at least an equimolar quantity of acid is employed relative to the imidazopyridine starting material. In general, a slight excess of hydrochloric acid is preferred.

After hydrochloric acid addition the mixture is cooled to a temperature in the range of about -15°C to about 10°C. The mixture, optionally seeded with 2-[2-methoxy-4-(methylsulfinyl)phenyl]-1(3)H-imidazo[4,5-c]pyridine hydrochloride dihydrate, is stirred for about 1 to about 24 hours at a temperature of about -15°C to about 10°C, preferably 5°C. The dihydrate will crystallize and can be recovered, if desired, using any of the standard isolation techniques previously described, preferably filtration.

If any of the above produced dihydrate crystals are washed after isolation they should be washed with a water/organic solvent mixture composed of an alcohol or ketone organic solvent combined with water in a ratio of about 4:1 to about 10:1 (v:v) organic solvent to water. Preferably, the wash mixture should contain the same organic solvent in about the same organic solvent to water ratio as the water/organic solvent mixture used to dissolve or slurry the imidazopyridine or monohydrate starting material.

Residual solvent remaining on the dihydrate product produced by any of the processes of this invention can be removed, if desired, by drying the product. Any conventional drying technique, such as air drying, vacuum drying, or forced air drying, may be used. If the residual solvent is removed by a drying technique which employs temperatures above ambient temperature, the drying temperature should be kept below about 40°C. A preferred drying temperature is about 30°C.

According to another aspect of the invention there is provided a method of treating a mammal, including a human, suffering from or susceptible to the conditions of asthma, thrombosis, hypertension, or heart failure, which comprises administering to said mammal a therapeutically effective amount of either 2-[2-methoxy-4-(methylsulfinyl)phenyl]-1(3)H-imidazo[4,5-c]pyridine hydrochloride monohydrate, its individual polymorphs, 2-[2-methoxy-4-(methylsulfinyl)phenyl]-1(3)H-imidazo[4,5-c]pyridine hydrochloride dihydrate, or a mixture thereof. The monohydrate and dihydrate compounds of the present invention are useful as effective positive inotropic agents which have minimal effects on blood pressure and heart rate. The compounds also possess vasodilation and anticoagulent activity. Furthermore, both hydrates are useful in treating any condition characterized by excessive release of slow reacting substances of anaphylaxis (SRS-A), including immediate-type hypersensitivity reactions such as asthma.

The monohydrate and dihydrate may be administered by various routes including the oral, rectal, transdermal, subcutaneous, intravenous, intramuscular, or intranasal routes. It is a special feature of these compounds that they are effective positive inotropic agents, vasodilators, or bronchodilators following oral administration.

The hydrate compounds of the invention are effective over a wide dosage range. For example, dosages per day will normally fall within the range of about 0.25 to 50 mg/kg. In the treatment of adult humans, the range of about 1 to 10 mg/kg, in single or divided doses, is preferred. However, it will be understood that the amount of the compound actually administered will be determined by a physician, in the light of the relevant circumstances including the condition to be treated, the chosen route of administration, the age, weight, and response of the individual patient, and the severity of the patient's symptoms, and therefore the above dosage ranges are not intended to limit the scope of the invention in any way.

2-[2-Methoxy-4-(methylsulfinyl)phenyl]-1(3)H-imidazo[4,5-c]pyridine hydrochloride monohydrate and the corresponding dihydrate can be formulated in a manner well known to those skilled in the pharmaceutical art of formulations. Accordingly, yet another aspect of the present invention are pharmaceutical formulations comprising as "active ingredient" either 2-[2-methoxy-4-(methylsulfinyl)phenyl]-1(3)H-imidazo[4,5-c]pyridine hydrochloride monohydrate, 2-[2-methoxy-4-(methylsulfinyl)phenyl]-1(3)H-imidazo[4,5-c]pyridine hydrochloride dihydrate, or a mixture thereof, in association with one or more pharmaceutically acceptable carriers or diluents therefor.

In making the formulations of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the composition can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders. The formulations of the invention may, also, as is well known in the art, be formulated so as to provide quick, sustained or delayed release of the active ingredient after administration to the patient.

Some examples of suitable carriers are lactose, dextrose, sucrose, sorbitol, mannitol, starches, gum

7

acacia, calcium phosphate, alginates, tragacanth, gelatin, calcium silicate, microcrystalline cellulose, polyvinylpyrrolidone, cellulose, water, syrup, methyl cellulose, methyl- and propyl-hydroxybenzoates, talc, magnesium stearate or mineral oil. The formulations can additionally include lubricating agents, wetting agents, emulsifying and suspending agents, preserving agents, sweetening agents or flavoring agents.

The formulations are preferably formulated in a unit dosage form, each dosage containing from about 1 to 500 mg, more usually about 2 to 100 mg, of the active ingredient. The term "unit dosage form" refers to physically discrete units suitable as unitary dosages for human subjects and other mammals, each unit containing a predetermined quantity of active material calcu lated to produce the desired therapeutic effect, in association with one or more pharmaceutical carriers or diluents.

The following Examples illustrate specific aspects of the present invention. The Examples are not intended to limit the scope of the present invention in any respect and should not be so construed.

The following Examples 1-3 all yield 2-[2-methoxy-4-(methylsulfinyl)phenyl]-1(3)H-imidazo[4,5-c]pyridine hydrochloride monohydrate.

## Example 1

## Polymorphic State I

A screw cap bottle (20 x 150 mm) was filled approximately 75% full with isopropanol. 2-(2-Methoxy-4-(methylsulfinyl)phenyl]-1(3)H-imidazo[4,5-c]pyridine hydrochloride was added in a quantity sufficient to form a slurry. The bottle was placed on a mechanical shaker for two hours and then placed in a dark room for several days. The solid phase was recovered by vacuum filtration and allowed to air dry at room temperature (22° C) overnight. Karl Fisher (5.4%) and x-ray analysis (shown below) confirmed that the recovered product was the monohydrate of polymorphic State I.

| d | $I/I_1$ |
|---|---|
| 10.07 | .35 |
| 6.37 | .11 |
| 5.86 | .22 |
| 5.68 | .04 |
| 4.97 | .48 |
| 4.55 | .05 |
| 4.43 | .02 |
| 4.25 | .13 |
| 4.19 | .03 |
| 3.96 | .36 |
| 3.60 | 1.00 |
| 3.33 | .49 |
| 2.89 | .36 |
| 2.34 | .14 |
| 2.12 | .06 |
| 2.03 | .06 |

## Example 2

## Polymorphic State II

To a 200 gallon tank containing 99 liters of glacial acetic acid were added 17.8 kg of 2-[2-methoxy-4-(methylthio)phenyl]-1(3)H-imidazo[4,5-c]pyridine. The resulting solution was cooled to about 15°C and 7.1 kg of a 35% (weight %) hydrogen peroxide solution were added at a rate such that the solution's temperature was maintained between 15° to 20°C. The reaction solution was stirred at ambient temperature (22°C) for about 20 hours, while monitoring the progress of the reaction by the high performance liquid chromatography (HPLC) assay discussed below.

The assay samples were dissolved in an internal standard solution consisting of 0.7 mg of 2-naphthalene sulfonic acid sodium salt per ml of an elution solvent comprised of 400 parts of acetonitrile, 100 parts of tetrahydrofuran, 21 parts of glacial acetic acid and 1479 parts of water. The column was eluted with the elution solvent listed above containing 2.2 g of 1-heptane sulfonic acid sodium salt. The column employed was a Whatman Partisil PXS 5/25 ODS. The detector had a wavelength of 320 nm, the column flow rate was 1.0 ml/min, the injection volume was 20 $\mu$l and the column temperature was ambient.

Once the HPLC assay indicated the reaction was complete, isopropanol (256 liters) was added and the resulting solution cooled to about 5°C and stirred at that temperature for 5 hours. 2-[2-Methoxy-4-(methylsulfinyl)phenyl]-1(3)H-imidazo[4,5-c]pyridine acetate crystallized and was recovered by filtration then washed with 33 liters of a cold 7.25:1 (v:v) isopropanol/acetic acid solution.

The acetate salt was dissolved in 58 liters of water. Activated carbon (2.0 kg) and concentrated hydrochloric acid (9 liters of a 38% by weight solution of hydrochloric acid in water) were added and the resulting slurry stirred for about 2 hours. The activated carbon was removed by filtration and washed with 279 liters of isopropanol.

Both the filtrate and the carbon wash solution were added to a 200 gallon tank which contained 156 liters of isopropanol. The mixture was cooled to about -5°C and stirred at that temperature for sixteen hours 2-[2-Methoxy-4-(methylsulfinyl)phenyl]-1(3)H-imidazo[4,5-c]pyridine hydrochloride crystallized and was recovered by filtration.

The isolated product was washed with 77 liters of a cold 8.8:1 isopropanol/water mixture, followed by 77 liters of isopropanol. The resulting solid was dried in a vacuum oven at about 50°C to provide 17.4 kg of 2-[2-methoxy-4-(methylsulfinyl)phenyl]-1(3)H-imidazo[4,5-c]pyridine hydrochloride monohydrate. Karl Fisher (6.56%) and x-ray analysis (shown below) confirmed that the recovered product was the monohydrate of polymorphic State II.

| d · | I/I$_1$ |
|---|---|
| 14.12 | .11 |
| 10.68 | .11 |
| 6.96 | .71 |
| 5.95 | .11 |
| 5.03 | .22 |
| 4.83 | .56 |
| 4.67 | .14 |
| 3.97 | .38 |
| 3.86 | .06 |
| 3.75 | .17 |
| 3.51 | 1.00 |
| 3.27 | .16 |
| 3.10 | .50 |
| 2.86 | .08 |
| 2.68 | .06 |
| 2.34 | .25 |
| 2.00 | .11 |

Example 3

Polymorphic State III

To a 200 gallon tank containing 90 liters of glacial acetic acid were added 16.3 kg of 2-[2-methoxy-4-(methylthio)phenyl]-1(3)H-imidazo[4,5-c]pyridine. The resulting solution was cooled to about 15°C and 6.5 kg of a 35% (weight %) hydrogen peroxide solution were added at a rate such that the solution's temperature was maintained between 15° to 20°C. The reaction solution was stirred at ambient temperature (22°C) for about 20 hours, while monitoring the progress of the reaction by the high performance liquid chromatography (HPLC) assay discussed in Example 2.

Once the HPLC assay indicated the reaction was complete, isopropanol (233 liters) was added and the resulting solution cooled to about 5°C and stirred at that temperature for 5 hours. 2-[2-Methoxy-4-(methylsulfinyl)phenyl]-1(3)H-imidazo[4,5-c]pyridine acetate crystallized and was recovered by filtration then washed with 30.2 liters of a cold 7.25:1 (v:v) isopropanol/acetic acid solution.

The acetate salt was dissolved in 53 liters of water. Activated carbon (2.0 kg) and concentrated hydrochloric acid (8.2 liters of a 38% by weight solution of hydrochloric acid in water) were added and the resulting slurry stirred for about 2 hours. The activated carbon was removed by filtration and washed with 254 liters of isopropanol.

The aqueous filtrate produced above was added to a 200 gallon tank. Cooling of the aqueous solution was started before either the carbon wash solution or any fresh isopropanol were added. As the tank contents cooled the carbon wash solution from above and 142 liters of fresh isopropanol were added. 2-[2-Methoxy-4-(methylsulfinyl)phenyl]-1(3)H-imidazo[4,5-c]pyridine hydrochloride crystallized before all of the carbon wash solution and fresh isopropanol could be added. After all of the carbon wash solution and fresh isopropanol were added cooling continued until the mixture temperature reached about -5°C. The mixture was stirred at about -5°C for sixteen hours and then the crystallized product was recovered by filtration.

The isolated product was washed with 69 liters of a cold 8.8:1 (v:v) isopropanol/water mixture, followed by 69 liters of isopropanol. The resulting solid was dried in a vacuum oven at about 45°C to provide 16.3 kg of a mixture of 2-[2-methoxy-4-(methylsulfinyl)phenyl]-1(3)H-imidazo[4,5-c]pyridine hydrochloride monohydrate and the corresponding dihydrate. Karl Fisher analysis (8.75%) established that the recovered crystals were a mixture of monohydrate and dihydrate forms.

The monohydrate and dihydrate mixture produced above was converted to pure monohydrate by dissolving the mixture in 65.4 liters of water. Isopropanol (491 liters) was added to the aqueous solution and the resulting mixture was stirred at ambient temperature (22°C) for one hour. The mixture was cooled to 5°C and stirred at that temperature for sixteen hours. 2-[2-Methoxy-4-(methylsulfinyl)phenyl]-1(3)H-imidazo-[4,5-c]pyridine hydrochloride monohydrate crystallized and was recovered by filtration.

The isolated monohydrate was washed with 86 liters of a cold 8.8:1 (v:v) isopropanol/water mixture, followed by 86 liters of isopropanol. The resulting solid was dried by pulling air through the recovered product for 18 hours, followed by drying in a vacuum oven at about 45°C. The dried product provided 12.9 kg of 2-[2-methoxy-4-(methylsulfinyl)phenyl]-1(3)H-imidazo[4,5-c]pyridine hydrochloride monohydrate. Karl Fisher (5.78%) and x-ray analysis (shown below) confirmed that the recovered product was the monohydrate of polymorphic State III.

| d | I/I$_1$ |
|---|---|
| 13.86 | .08 |
| 10.61 | .11 |
| 10.04 | .06 |
| 7.60 | .04 |
| 6.93 | .52 |
| 6.37 | .07 |
| 5.89 | .25 |
| 5.30 | .09 |
| 4.98 | .30 |
| 4.81 | .66 |
| 4.64 | .25 |
| 4.25 | .20 |
| 3.96 | 1.00 |
| 3.74 | .32 |
| 3.60 | .80 |
| 3.50 | 1.00 |
| 3.34 | .33 |
| 3.17 | .16 |
| 3.09 | .89 |
| 2.86 | .13 |
| 2.67 | .15 |

The following Examples 4-10 all yield 2-[2-methoxy-4-(methylsulfinyl)phenyl]-1(3)H-imidazo[4,5-c]-pyridine hydrochloride dihydrate.

## Example 4

### Coverting Monohydrate to Dihydrate

#### 4A) Seeded Mixture

One hundred grams of 2-[2-methoxy-4-(methylsulfinyl)phenyl]-1(3)H-imidazo[4,5-c]pyridine hydrochloride monohydrate were dissolved in 200 ml of hot (42°C) water. The solution was divided into two approximately equal portions and hot isopropanol (750 ml at 42°C) was added to each over a period of five minutes. One mixture was seeded with authentic 2-[2-methoxy-4-(methylsulfinyl)phenyl]-1(3)H-imidazo[4,5-c]pyridine hydrochloride dihydrate. The seeded mixture was slowly cooled from 42°C to room temperature (22°C) over a period of about 90 minutes. 2-[2-Methoxy-4-(methylsulfinyl)phenyl]-1(3)H-imidazo[4,5-c]-pyridine hydrochloride dihydrate crystallized and was recovered by vacuum filtration. The crystals were vacuum dried overnight at 30°C and then evaluated by Karl Fisher analysis The seeded mixture produced crystals which assayed 10.1% water. X-ray analysis (shown below) confirmed the product as the dihydrate.

| d | I/I$_1$ |
|---|---|
| 13.05 | .05 |
| 11.67 | .24 |
| 9.23 | .11 |
| 7.60 | .05 |
| 7.27 | .25 |
| 6.57 | .16 |
| 5.86 | .45 |
| 5.25 | .09 |
| 4.88 | .16 |
| 4.15 | .28 |
| 3.90 | 1.00 |
| 3.62 | .20 |
| 3.49 | .56 |
| 3.35 | .07 |
| 3.24 | .46 |
| 3.18 | .42 |
| 3.07 | .08 |
| 2.99 | .10 |
| 2.93 | .05 |
| 2.62 | .18 |

4B) Non-seeded Mixture

The remaining mixture prepared above was left unseeded. 2-[2-Methoxy-4-(methylsulfinyl)phenyl]-1(3)-H-imidazo[4,5-c]pyridine hydrochloride dihydrate crystallized and was recovered per the procedure described above. The crystals assayed 9.8% water by Karl Fisher analysis and had the same x-ray diffraction pattern as in 4A above.

Example 5

Converting Monohydrate to Dihydrate

5A) Air Drying

One hundred grams of 2-[2-methoxy-4-(methylsulfinyl)phenyl]-1(3)H-imidazo[4,5-c]pyridine hydrochloride monohydrate were dissolved in 200 ml of 50°C water. Isopropanol (1500 ml at 50°C) was added over a period of ten minutes. The mixture was cooled to room temperature (22°C) over a period of about 105 minutes. The dihydrate crystallized and was recovered by vacuum filtration to provide 89.4 g of product material. The recovered product was divided into two lots, one of which was allowed to air dry at room temperature (22°C). The air dried product assayed 9.9% water by Karl Fisher analysis and had the same x-ray diffraction pattern as set forth in 4A above.

5B) Vacuum Drying

The second lot from above was dried in a vacuum oven at 30°C to produce a product which assayed 9.9% water by Karl Fisher analysis. This product also had the x-ray diffraction pattern set forth in 4A above.

## Example 6

### Converting Monohydrate to Dihydrate

Ten grams of the monohydrate were dissolved in 20 ml of room temperature (22°C) water. Isopropanol (150 ml) was added, with stirring, over a five minute period. The mixture was stirred for about an hour while 2-[2-methoxy-4-(methylsulfinyl)phenyl]-1(3)H-imidazo[4,5-c]pyridine hydrochloride dihydrate precipitated. The precipitate was recovered by vacuum filtration and dried in a vacuum oven at 30°C overnight. After drying, the product assayed 10.0% water by Karl Fisher analysis. X-ray analysis confirmed that the product had the same x-ray diffraction pattern as disclosed in Example 4A.

## Example 7

### Converting Monohydrate to Dihydrate

Ten grams of the monohydrate were dissolved in 40 ml of a 2:1 denatured ethanol/water mixture. Additional denatured ethanol (80 ml) was added. The mixture was seeded with 2-[2-methoxy-4-(methylsulfinyl)phenyl]-1(3)H-imidazo[4,5-c]pyridine hydrochloride dihydrate and stirred for about 2 hours at room temperature (22°C). The dihydrate began to crystallize while the mixture was stirring.

To increase the yield the mixture was slowly cooled to about 0°C and stirred for an additional $1\frac{1}{2}$ hours. The precipitate was recovered by vacuum filtration and washed with 15 ml of a cold 1:1 denatured ethanol/water mixture, followed by an additional 15 ml of cold denatured ethanol. Karl Fisher analysis of the recovered solid indicated a water content of 9.8%. X-ray analysis confirmed that the product had the same x-ray diffraction pattern as in Example 4A.

## Example 8

### Converting Monohydrate to Dihydrate

8A) 50% Isopropanol Mixture

Ten grams of monohydrate were placed in a screw cap bottle and 37.5 ml of a 50% (volume % isopropanol) isopropanol/water mixture were added in order to form a slurry. The bottle was agitated on a rotating dissolution apparatus for one hour. After one hour the solids were recovered by vacuum filtration and allowed to air dry to provide a product which assayed 10.3% water by Karl Fisher analysis.

8B) 75% Isopropanol Mixture

The above procedure was repeated with the exception that 62.5 ml of a 75% (volume % isopropanol) isopropanol/water mixture were used. The resulting solids assayed 10.6% water by Karl Fisher analysis.

8C) 85% Isopropanol Mixture

The above procedure was repeated with the exception that 62.5 ml of an 85% (volume % isopropanol) isopropanol/water mixture were used. The resulting solids assayed 10.4% water by Karl Fisher analysis.


8D) 95% Isopropanol Mixture

The procedure above was repeated with the exception that 50.0 ml of a 95% (volume % isopropanol) isopropanol/water mixture were used. The resulting solids assayed 10.8% water by Karl Fisher analysis X-ray analysis of the recovered solids confirmed that the solids had the same x-ray diffraction pattern as described in Example 4A.


## Example 9


### Converting Monohydrate to Dihydrate


9A) 50% Acetone Mixture

An acetone/water mixture was prepared which contained 50% (volume %) acetone. The mixture was placed in a screw cap bottle and monohydrate was added in a quantity sufficient to form a slurry. The bottle was placed on a mechanical shaker for 47 hours. After agitation the solids were recoverd by vacuum filtration Karl Fisher analysis indicated the solids were 11.1% water.


9B) 75% Acetone Mixture

The above procedure was repeated with the exception that a 75% (volume % acetone) acetone/water mixture was used. Karl Fisher analysis indicated a water content of 9.8%.


9C) 80% Acetone Mixture

The above procedure was repeated with the exception that an 80% (volume % acetone) acetone/water mixture was used. Karl Fisher analysis indicated a water content of 10.0%.


9D) 85% Acetone Mixture

The above procedure was repeated with the exception that an 85% (volume % acetone) acetone/water mixture was used. The recovered solids, as indicated by Karl Fisher analysis, had a water content of 9.8%.


9E) 90% Acetone Mixture

The above procedure was repeated with the exception that a 90% (volume % acetone) acetone/water mixture was used. This solution produced solids which, by Karl Fisher analysis, were 10.0% water.


9F) 95% Acetone Mixture

The above procedure was repeated with the exception that a 95% (volume % acetone) acetone/water mixture was used. Karl Fisher analysis indicated a water content of 10.0%.


## Example 10

Converting Monohydrate to Dihydrate

A sample of monohydrate was placed in a chamber where it was exposed to an air atmosphere which was kept at 79% relative humidity and room temperature (22° C). After exposure to the air atmosphere for two weeks the sample was removed and assayed by X-ray analysis. The sample had the same x-ray diffraction pattern as in Example 4A.

Example 11

Directly Producing the Dihydrate from 2-[2-Methoxy-4-(methylsulfinyl)phenyl]-1(3)H-imidazo[4,5-c]-pyridine

2-[2-Methoxy-4-(methylsulfinyl)phenyl]-1(3)H-imidazo[4,5-c]pyridine acetate (7.4 g) was dissolved in 26 ml of water. Activated carbon (0.52 g) was added and the mixture stirred for about one hour. The activated carbon was removed by filtration and washed with 5 ml of water and 50 ml of isopropanol.

The filtrate and carbon wash solutions were added to a 500 ml, 3-neck flask, which contained 160 ml of isopropanol. Concentrated hydrochloric acid (3.3 ml of a 38% by weight solution of hydrochloric acid) was added and the mixture seeded with 2-[2-methoxy-4-(methyl sulfinyl)phenyl]-1(3)H-imidazo[4,5-c]pyridine hydrochloride dihydrate then cooled to about 5° C. The mixture was stirred for 3 hours at 5° C and the resulting precipitate recovered by filtration. The recovered precipitate was washed with 25 ml of a chilled 7:1 (v:v) isopropanol/water mixture and then dried in a vacuum oven at 40° C to provide 3.52 g of 2-[2-methoxy-4-(methylsulfinyl)phenyl]-1(3)H-imidazo[4,5-c]pyridine hydrochloride dihydrate. X-ray analysis confirmed that the product had the same x-ray diffraction pattern as described in Example 4A.

The following formulation examples may employ as active ingredient 2-[2-methoxy-4-(methylsulfinyl)-phenyl]-1(3)H-imidazo[4,5-c]pyridine hydrochloride monohydrate, a monohydrate of polymorphic State I, a monohydrate of polymorphic State II, a monohydrate of polymorphic State III, 2-[2-methoxy-4-(methylsulfinyl)phenyl]-1(3)H-imidazo[4,5-c]pyridine hydrochloride dihydrate, a crystalline dihydrate having the x-ray diffraction pattern set forth in Table 4, or a mixture of any of the above. The monohydrate, the various polymorphs thereof, the dihydrate, the crystalline dihydrate having the x-ray diffraction pattern set forth in Table 4, or a mixture of any of the above, are referred to in the following formulations as "Active Ingredient".

Examples 12-16

Hard gelatin capsules are prepared using the following ingredients:

Example 12

|  | Quantity (mg/capsule) |
| --- | --- |
| Active Ingredient | 2.55 |
| Starch Powder, N.F. | 116.00 |
| Silicone Fluid, 350 centistokes | 1.74 |
| Starch Flowable Powder | 229.06 |

Example 13

|  | Quantity (mg/capsule) |
|---|---|
| Active Ingredient | 5.10 |
| Starch Powder, N.F. | 113.00 |
| Silicone Fluid, 350 centistokes | 1.70 |
| Starch Flowable Powder | 223.90 |

Example 14

|  | Quantity (mg/capsule) |
|---|---|
| Active Ingredient | 10.20 |
| Starch Powder, N.F. | 109.00 |
| Silicone Fluid, 350 centistokes | 1.64 |
| Starch Flowable Powder | 216.56 |

Example 15

|  | Quantity (mg/capsule) |
|---|---|
| Active Ingredient | 25.50 |
| Starch Powder, N.F. | 98.00 |
| Silicone Fluid, 350 centistokes | 1.45 |
| Starch Flowable Powder | 193.55 |

Example 16

|  | Quantity (mg/capsule) |
|---|---|
| Active Ingredient | 51.00 |
| Starch Powder, N.F. | 82.00 |
| Silicone Fluid, 350 centistokes | 1.25 |
| Starch Flowable Powder | 162.75 |

The above ingredients in Examples 11-15 are mixed and filled into hard gelatin capsules in the quantities described above.

Examples 17 and 18

16

Tablet formulas are prepared using the ingredients below:

### Example 17

|  | Quantity (mg/tablet) |
|---|---|
| Active Ingredient | 250 |
| Cellulose, microcrystalline | 400 |
| Silicon dioxide, fumed | 10 |
| Stearic acid | 5 |

The components are blended and compressed to form tablets each weighing 665 mg.

### Example 18

|  | Quantity (mg/tablet) |
|---|---|
| Active Ingredient | 60 |
| Starch Powder, N.F. | 45 |
| Microcrystalline cellulose | 35 |
| Polyvinylpyrrolidone (as 10% solution in water) | 4 |
| Sodium carboxymethyl starch | 4.5 |
| Magnesium stearate | 0.5 |
| Talc | 1 |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

### Example 19

An aerosol solution is prepared containing the following components:

|  | Weight % |
|---|---|
| Active Ingredient | 0.25 |
| Ethanol | 29.75 |
| Propellant 22 (Chlorodifluoromethane) | 70.00 |

The active compound is mixed with ethanol and the mixture added to a portion of the propellant 22, cooled to -30°C and transferred to a filling device. The required amount is then fed to a stainless steel container and diluted further with the remainder of the propellant. The valve units are then fitted to the container.

17

EP 0 322 149 A2

## Example 20

Suppositories each containing 225 mg of active ingredient are made as follows:

| Active Ingredient | 225 mg |
|---|---|
| Saturated fatty acid glycerides | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

## Example 21

Suspensions each containing 50 mg of medicament per 5 ml dose are made as follows:

| Active Ingredient | 50 mg |
|---|---|
| Sodium carboxymethyl cellulose | 50 mg |
| Syrup | 1.25 ml |
| Benzoic acid solution | 0.10 ml |
| Flavor | q.v. |
| Color | q.v. |
| Purified water to | 5 ml |

The medicament is passed through a No. 45 mesh U.S. sieve and mixed with the sodium carboxymethylcellulose and syrup to form a smooth paste. The benzoic acid solution, flavor and color are diluted with some of the water and added, with stirring. Sufficient water is then added to produce the required volume.

## Example 22

A sustained release tablet is prepared containing the following components:

| | Quantity (mg/tablet) |
|---|---|
| Active Ingredient | 30.8 |
| Methocel E4M | 212.5 |
| Lactose, spray dried | 193.36 |
| Dextrates | 48.34 |
| Magnesium stearate | 5.0 |
| Stearic acid powder | 10.0 |

The components are blended and compressed to form sustained release tablets weighing 500.0 mg.

## Claims

1. A hydrate of 2[2-methoxy-4-(methylsulfinyl)phenyl]-1(3)H-imidazo[4,5-c]pyridine hydrochloride, wherein the hydrate is selected from the group consisting of the monohydrate or the dihydrate.

18

2. A hydrate of Claim 1 which is 2-[2-methoxy-4-(methylsulfinyl)phenyl]-1(3)H-imidazo[4,5-c]pyridine hydrochloride monohydrate.

3. A crystalline compound of Claim 2 which has the following x-ray powder diffraction obtained with nickel-filtered copper radiation of $\lambda = 1.54056\text{Å}$, wherein "d" represents the interplanar spacing and "$I/I_1$" the relative intensity:

| d | $I/I_1$ |
|---|---|
| 10.07 | .35 |
| 6.37 | .11 |
| 5.86 | .22 |
| 5.68 | .04 |
| 4.97 | .48 |
| 4.55 | .05 |
| 4.43 | .02 |
| 4.25 | .13 |
| 4.19 | .03 |
| 3.96 | .36 |
| 3.60 | 1.00 |
| 3.33 | .49 |
| 2.89 | .36 |
| 2.34 | .14 |
| 2.12 | .06 |
| 2.03 | .06 |

4. A crystalline compound of Claim 2 which has the following x-ray powder diffraction obtained with nickel-filtered copper radiation of $\lambda = 1.54056\text{Å}$, wherein "d" represents the interplanar spacing and "$I/I_1$" the relative intensity:

| d | $I/I_1$ |
|---|---|
| 14.12 | .11 |
| 10.68 | .11 |
| 6.96 | .71 |
| 5.95 | .11 |
| 5.03 | .22 |
| 4.83 | .56 |
| 4.67 | .14 |
| 3.97 | .38 |
| 3.86 | .06 |
| 3.75 | .17 |
| 3.51 | 1.00 |
| 3.27 | .16 |
| 3.10 | .50 |
| 2.86 | .08 |
| 2.68 | .06 |
| 2.34 | .25 |
| 2.00 | .11 |

5. A crystalline compound of Claim 2 which has the following x-ray powder diffraction obtained with nickel-filtered copper radiation of $\lambda = 1.54056\text{Å}$, wherein "d" represents the interplanar spacing and "$I/I_1$" the relative intensity:

19

| d | $I/I_1$ |
|---|---|
| 13.86 | .08 |
| 10.61 | .11 |
| 10.04 | .06 |
| 7.60 | .04 |
| 6.93 | .52 |
| 6.37 | .07 |
| 5.89 | .25 |
| 5.30 | .09 |
| 4.98 | .30 |
| 4.81 | .66 |
| 4.64 | .25 |
| 4.25 | .20 |
| 3.96 | 1.00 |
| 3.74 | .32 |
| 3.60 | .80 |
| 3.50 | 1.00 |
| 3.34 | .33 |
| 3.17 | .16 |
| 3.09 | .89 |
| 2.86 | .13 |
| 2.67 | .15 |

6. A hydrate of Claim 1 which is 2-[2-methoxy-4-(methylsulfinyl)phenyl]-1(3)H-imidazo[4,5-c]pyridine hydrochloride dihydrate.

7. A crystalline compound of Claim 6 which has the following x-ray powder diffraction obtained with nickel-filtered copper radiation of $\lambda$ = 1.54056Å, wherein "d" represents the interplanar spacing and "$I/I_1$" the relative intensity:

| d | $I/I_1$ |
|---|---|
| 13.05 | .05 |
| 11.67 | .24 |
| 9.23 | .11 |
| 7.60 | .05 |
| 7.27 | .25 |
| 6.57 | .16 |
| 5.86 | .45 |
| 5.25 | .09 |
| 4.88 | .16 |
| 4.15 | .28 |
| 3.90 | 1.00 |
| 3.62 | .20 |
| 3.49 | .56 |
| 3.35 | .07 |
| 3.24 | .46 |
| 3.18 | .42 |
| 3.07 | .08 |
| 2.99 | .10 |
| 2.93 | .05 |
| 2.62 | .18 |

8. A hydrate of 2-[2-methoxy-4-(methylsulfinyl)phenyl]-1(3)H-imidazo[4,5-c]pyridine hydrochloride as claimed in any one of Claims 1 to 7, or a mixture thereof, for use in treating a mammal suffering from or susceptible to the conditions of asthma, thrombosis, hypertension or heart failure.

9. A pharmaceutical formulation comprising a hydrate of 2-[2-methoxy-4-(methylsulfinyl)phenyl]-1(3)H-imidazo[4,5-c]pyridine hydrochloride as claimed in any one of Claims 1 to 7, or a mixture thereof, in association with one or more pharmaceutically acceptable carriers or diluents therefor.

10. A process for preparing a hydrate of 2-[2-methoxy-4-(methylsulfinyl)phenyl]-1(3)H-imidazo[4,5-c]-pyridine hydrochloride as claimed in any one of Claims 1 to 7, which comprises

a) preparing a slurry consisting of 2-[2-methoxy-4-(methylsulfinyl)phenyl]-1(3)H-imidazo[4,5-c]pyridine hydrochloride mixed with an alcohol solvent, and intimately mixing the liquid and solid phases of the slurry to provide a monohydrate hydrate;

b) substantially dissolving 2-[2-methoxy-4-(methylsulfinyl)phenyl]-1(3)H-imidazo[4,5-c]pyridine hydrochloride in water, adding an alcohol counter-solvent, and cooling the alcohol/water mixture as rapidly as possible to a temperature in the range of from about -20°C to about 10°C to provide a monohydrate hydrate;

c) substantially dissolving a mixture of 2-[2-methoxy-4-(methylsulfinyl)phenyl]-1(3)H-imidazo[4,5-c]-pyridine hydrochloride monohydrate and the corresponding dihydrate in water, adding an alcohol counter-solvent, and cooling the alcohol/water mixture as rapidly as possible to a temperature in the range of from about -20°C to about 10°C to provide a hydrate which is totally monohydrate hydrate;

d) substantially dissolving 2-[2-methoxy-4-(methylsulfinyl)phenyl]-1(3)H-imidazo[4,5-c]pyridine hydrochloride monohydrate in water, or a water/alcohol or ketone organic solvent mixture, and adding additional alcohol or ketone organic solvent to provide a dihydrate hydrate;

e) preparing a slurry consisting of 2-[2-methoxy-4-(methylsulfinyl)phenyl]-1(3)H-imidazo[4,5-c]pyridine hydrochloride monohydrate mixed with water and an alcohol or ketone organic solvent, and contacting the solid phase with the liquid phase to provide a dihydrate hydrate;

f) exposing 2-[2-methoxy-4-(methylsulfinyl)phenyl]-1(3)H-imidazo[4,5-c]pyridine hydrochloride monohydrate to a gas which has a relative humidity of about 50% relative humidity to about 95% relative humidity and a temperature in the range of from about 15°C to about 60°C to provide a dihydrate hydrate; or

g) substantially dissolving 2-[2-methoxy-4-(methylsulfinyl)phenyl]-1(3)H-imidazo[4,5-c]pyridine, or a weak acid salt thereof, in a water/alcohol or ketone organic solvent mixture, adding hydrochloric acid, and cooling the mixture to a temperature in the range of from about -15°C to about 10°C to provide a dihydrate hydrate.

Claims for the following Contracting States : GR;ES

1. A process for preparing a hydrate of 2-[2-methoxy-4-(methylsulfinyl)phenyl]-1(3)H-imidazo[4,5-c]-pyridine hydrochloride, wherein the hydrate is selected from the group consisting of the monohydrate or dihydrate, which comprises

a) preparing a slurry consisting of 2-[2-methoxy-4-(methylsulfinyl)phenyl]-1(3)H-imidazo[4,5-c]pyridine hydrochloride mixed with an alcohol solvent, and intimately mixing the liquid and solid phases of the slurry to provide a monohydrate hydrate;

b) substantially dissolving 2-[2-methoxy-4-(methylsulfinyl)phenyl]-1(3)H-imidazo[4,5-c]pyridine hydrochloride in water, adding an alcohol counter-solvent, and cooling the alcohol/water mixture as rapidly as possible to a temperature in the range of from about -20°C to about 10°C to provide a monohydrate hydrate;

c) substantially dissolving a mixture of 2-[2-methoxy-4-(methylsulfinyl)phenyl]-1(3)H-imidazo[4,5-c]-pyridine hydrochloride monohydrate and the corresponding dihydrate in water, adding an alcohol counter-solvent, and cooling the alcohol/water mixture as rapidly as possible to a temperature in the range of from about -20°C to about 10°C to provide a hydrate which is totally monohydrate hydrate;

d) substantially dissolving 2-[2-methoxy-4-(methylsulfinyl)phenyl]-1(3)H-imidazo[4,5-c]pyridine hydrochloride monohydrate in water, or a water/alcohol or ketone organic solvent mixture, and adding additional alcohol or ketone organic solvent to provide a dihydrate hydrate;

e) preparing a slurry consisting of 2-[2-methoxy-4-(methylsulfinyl)phenyl]-1(3)H-imidazo[4,5-c]pyridine hydrochloride monohydrate mixed with water and an alcohol or ketone organic solvent, and contacting the solid phase with the liquid phase to provide a dihydrate hydrate;

f) exposing 2-[2-methoxy-4-(methylsulfinyl)phenyl]-1(3)H-imidazo[4,5-c]pyridine hydrochloride monohydrate to a gas which has a relative humidity of about 50% relative humidity to about 95% relative humidity and a temperature in the range of from about 15°C to about 60°C to provide a dihydrate hydrate; or

g) substantially dissolving 2-[2-methoxy-4-(methylsulfinyl)phenyl]-1(3)H-imidazo[4,5-c]pyridine, or a weak acid salt thereof, in a water/alcohol or ketone organic solvent mixture, adding hydrochloric acid, and cooling the mixture to a temperature in the range of from about -15°C to about 10°C to provide a dihydrate hydrate.

2. A process according to Claim 1 for preparing 2-[2-methoxy-4-(methylsulfinyl)phenyl]-1(3)H-imidazo-[4,5-c]pyridine hydrochloride dihydrate which comprises

a) substantially dissolving 2-[2-methoxy-4-(methylsulfinyl)phenyl]-1(3)H-imidazo[4,5-c]pyridine hydrochloride monohydrate in water, or a water/alcohol or ketone organic solvent mixture, and adding additional alcohol or ketone organic solvent to provide a dihydrate hydrate;

b) preparing a slurry consisting of 2-[2-methoxy-4-(methylsulfinyl)phenyl]-1(3)H-imidazo[4,5-c]pyridine hydrochloride monohydrate mixed with water and an alcohol or ketone organic solvent, and contacting the solid phase with the liquid phase to provide a dihydrate hydrate;

c) exposing 2-[2-methoxy-4-(methylsulfinyl)phenyl]-1(3)H-imidazo[4,5-c]pyridine hydrochloride monohydrate to a gas which has a relative humidity of about 50% relative humidity to about 95% relative humidity and a temperature in the range of from about 15°C to about 60°C to provide a dihydrate hydrate; or

d) substantially dissolving 2-[2-methoxy-4-(methylsulfinyl)phenyl]-1(3)H-imidazo[4,5-c]pyridine, or a weak acid salt thereof, in a water/alcohol or ketone organic solvent mixture, adding hydrochloric acid, and cooling the mixture to a temperature in the range of from about -15°C to about 10°C to provide a dihydrate hydrate.

3. A process according to Claim 2 for preparing 2-[2-methoxy-4-(methylsulfinyl)phenyl]-1(3)H-imidazo-[4,5-c]pyridine hydrochloride dihydrate which comprises

a) substantially dissolving 2-[2-methoxy-4-(methylsulfinyl)phenyl]-1(3)H-imidazo[4,5-c]pyridine hydrochloride monohydrate in water, or a water/alcohol or ketone organic solvent mixture, and adding additional alcohol or ketone organic solvent to provide a dihydrate hydrate;

b) preparing a slurry consisting of 2-[2-methoxy-4-(methylsulfinyl)phenyl]-1(3)H-imidazo[4,5-c]pyridine hydrochloride monohydrate mixed with water and an alcohol or ketone organic solvent, and contacting the solid phase with the liquid phase to provide a dihydrate hydrate; or

c) substantially dissolving 2-[2-methoxy-4-(methylsulfinyl)phenyl]-1(3)H-imidazo[4,5-c]pyridine, or a weak acid salt thereof, in a water/alcohol or ketone organic solvent mixture, adding hydrochloric acid, and cooling the mixture to a temperature in the range of from about -15°C to about 10°C to provide a dihydrate hydrate.

4. A process according to Claim 3 which comprises the additional step of collecting the dihydrate.

5. A process according to Claim 4 which comprises the additional step of washing the collected dihydrate with a water/alcohol or ketone organic solvent mixture.

6. A process according to Claim 4 which comprises the additional step of drying the collected dihydrate using a drying temperature which is less than about 40°C.

7. A process according to Claim 2 for preparing 2-[2-methoxy-4-(methylsulfinyl)phenyl]-1(3)H-imidazo-[4,5-c]pyridine hydrochloride dihydrate, wherein the dihydrate has the following x-ray powder diffraction obtained with nickel-filtered copper radiation of $\lambda$ = 1.54056Å, wherein "d" represents the interplanar spacing and "$I/I_1$" the relative intensity:

| d | $I/I_1$ |
|---|---|
| 13.05 | .05 |
| 11.67 | .24 |
| 9.23 | .11 |
| 7.60 | .05 |
| 7.27 | .25 |
| 6.57 | .16 |
| 5.86 | .45 |
| 5.25 | .09 |
| 4.88 | .16 |
| 4.15 | .28 |
| 3.90 | 1.00 |
| 3.62 | .20 |
| 3.49 | .56 |
| 3.35 | .07 |
| 3.24 | .46 |
| 3.18 | .42 |
| 3.07 | .08 |
| 2.99 | .10 |
| 2.93 | .05 |
| 2.62 | .18 |

8. A process according to Claim 1 for preparing 2-[2-methoxy-4-(methylsulfinyl)phenyl]-1(3)H-imidazo-[4,5-c]pyridine hydrochloride monohydrate, wherein the monohydrate has the following x-ray powder diffraction obtained with nickel-filtered copper radiation of $\lambda$ = 1.54056Å, wherein "d" represents the interplanar spacing and "$I/I_1$" the relative intensity:

| d | $I/I_1$ |
|---|---|
| 10.07 | .35 |
| 6.37 | .11 |
| 5.86 | .22 |
| 5.68 | .04 |
| 4.97 | .48 |
| 4.55 | .05 |
| 4.43 | .02 |
| 4.25 | .13 |
| 4.19 | .03 |
| 3.96 | .36 |
| 3.60 | 1.00 |
| 3.33 | .49 |
| 2.89 | .36 |
| 2.34 | .14 |
| 2.12 | .06 |
| 2.03 | .06 |

which comprises preparing a slurry consisting of 2-[2-methoxy-4-(methylsulfinyl)phenyl]-1(3)H-imidazo[4,5-c]pyridine hydrochloride mixed with an alcohol solvent and intimately mixing the liquid and solid phases of the slurry.

9. A process according to Claim 1 for preparing 2-[2-methoxy-4-(methylsulfinyl)phenyl]-1(3)H-imidazo-[4,5-c]pyridine hydrochloride monohydrate, wherein the monohydrate has the following x-ray powder diffraction obtained with nickel-filtered copper radiation of $\lambda$ = 1.54056Å, wherein "d" represents the interplanar spacing and "$I/I_1$" the relative intensity:

23

| d | I/I$_1$ |
|---|---|
| 14.12 | .11 |
| 10.68 | .11 |
| 6.96 | .71 |
| 5.95 | .11 |
| 5.03 | .22 |
| 4.83 | .56 |
| 4.67 | .14 |
| 3.97 | .38 |
| 3.86 | .06 |
| 3.75 | .17 |
| 3.51 | 1.00 |
| 3.27 | .16 |
| 3.10 | .50 |
| 2.86 | .08 |
| 2.68 | .06 |
| 2.34 | .25 |
| 2.00 | .11 |

which comprises substantially dissolving 2-[2-methoxy-4-(methylsulfinyl)phenyl]-1(3)H-imidazo[4,5-c]-pyridine hydrochloride in water, adding an alcohol counter-solvent, and cooling the alcohol/water mixture as rapidly as possible to a temperature in the range of from about -20°C to about 10°C.

10. A pharmaceutical formulation comprising a hydrate of 2-[2-methoxy-4-(methylsulfinyl)phenyl]-1(3)H-imidazo[4,5-c]pyridine hydrochloride, as defined in any one of Claims 1 to 9, or a mixture thereof, in association with one or more pharmaceutically acceptable carriers or diluents therefor.

11. A process for preparing a pharmaceutical formulation comprising admixing a hydrate of 2-[2-methoxy-4-(methylsulfinyl)phenyl]-1(3)H-imidazo[4,5-c]pyridine hydrochloride as defined in any one of Claims 1 to 9, or a mixture thereof, with one or more pharmaceutically acceptable carriers or diluents.